(19) **Europäisches Patentamt**
**European Patent Office**
**Office européen des brevets**

(11) **EP 4 700 123 A2**

(12) **EUROPEAN PATENT APPLICATION**
published in accordance with Art. 153(4) EPC

(43) Date of publication:
**25.02.2026 Bulletin 2026/09**

(21) Application number: **24792185.1**

(22) Date of filing: **19.06.2024**

(51) International Patent Classification (IPC):
**C12N 15/10** (2006.01)    **C12N 13/00** (2006.01)
**C12N 15/01** (2006.01)    **G01N 21/64** (2006.01)
**G01N 15/14** (2024.01)    **C12P 19/34** (2006.01)

(86) International application number:
**PCT/CN2024/100136**

(87) International publication number:
**WO 2024/217602 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.04.2023 CN 202310427005**

(71) Applicant: **Angel Yeast Co., Ltd**
**Yichang, Hubei 443003 (CN)**

(72) Inventors:
• **WU, Yexu**
**Yichang, Hubei 443003 (CN)**

• **XIAO, Minghua**
**Yichang, Hubei 443003 (CN)**
• **HU, Yue**
**Yichang, Hubei 443003 (CN)**
• **ZHANG, Yan**
**Yichang, Hubei 443003 (CN)**
• **LEI, Senlin**
**Yichang, Hubei 443003 (CN)**
• **HUANG, Xingfa**
**Yichang, Hubei 443003 (CN)**

(74) Representative: **karo IP**
**Patentanwälte PartG mbB**
**Steinstraße 16-18**
**40212 Düsseldorf (DE)**

(54) **METHOD FOR HIGH-THROUGHPUT AND RAPID BREEDING OF HIGH-RNA YEAST**

(57)    A method for the high-throughput and rapid breeding of high-RNA yeast. The method for the high-throughput breeding of high-RNA yeast comprises the following steps: (1) establishing a microbial mutant library; (2) recovering bacterial cells obtained in step (1); (3) subjecting the mutant library to adaptive evolution; and (4) carrying out high-throughput screening on the evolutionary library to obtain a high-RNA yeast. By means of the provided method, the high-RNA yeast strain can be efficiently screened out, the dominant strain is rapidly bred, and the RNA content of the screened dominant strain can reach 15% or more.

**EP 4 700 123 A2**

## Description

### Technical Field

The present invention relates to a bioengineering technology, and more particularly, to a method for high-throughput screening of a microbial strain.

### Background Art

Ribonucleic acid (RNA) is an important biological macromolecule, which is widely used in food flavoring, nutrition and health care, biomedicine and other fields. At present, the industrial production of RNA mainly includes extraction from purely cultured *Candida oleophila, Saccharomyces cerevisiae* or beer yeast paste. *Saccharomyces cerevisiae* does not produce any toxins, is a microorganism generally recognized as safe (GRAS), has a fast growth and passage rate, is easy to culture, has a nutritional value, and is recognized as the most ideal source of RNA.

Due to the low RNA content of *Saccharomyces cerevisiae,* which averages 6%-8%, the costs of RNA, nucleotides and other products derived from *Saccharomyces cerevisiae* are high. In order to reduce the production cost and improve the product competitiveness, it is necessary to further increase the RNA content of yeast strains.

### Summary of the Invention

The traditional screening method adopted in the prior art is difficult to achieve large-scale and efficient screening, and cumbersome in operation. In view of the problems existing in the prior art, the present invention provides a method for high-throughput and rapid breeding of a high-RNA yeast.

The present invention provides a method for high-throughput and rapid breeding of a high-RNA yeast. The method includes the following steps:

(1) establishing a microbial mutant library;
(2) recovering bacterial cells obtained in step (1);
(3) subjecting the mutant library to adaptive evolution; and
(4) carrying out high-throughput screening on the evolutionary library to obtain a high-RNA yeast.

Preferably, in step (1), the establishing the microbial mutant library is implemented by using chemical mutagenesis, physical mutagenesis or a combination of chemical mutagenesis and physical mutagenesis.

More preferably, a chemical mutagen is one or a combination of more of ethyl methanesulfonate, diethyl sulfate, and sodium azide; and

further preferably, an action concentration of the chemical mutagens is 1-6% (w/v). The percentage content of the action concentration of the chemical mutagen is calculated in w/v, and specifically refers that a mass of the chemical mutagen contained in every 100 mL of chemical mutagen solution is 1-6 g, wherein preferably, a volume ratio of the chemical mutagen relative to the yeast suspensions is (0.5-2.5): 1, more preferably, 1: 1.

More preferably, the action time is 0.5-2 h.

More preferably, the physical mutagenesis is implemented by using one or a combination of more of ultraviolet radiation and atmospheric and room temperature plasma;

more preferably, the physical mutagenesis time is 30 s-90 s;
more preferably, the atmospheric and room temperature plasma has a power of 80-120 W, and an air flow rate of 8-15 L/min; more preferably, the atmospheric and room temperature plasma has a power of 100 W, and an air flow rate of 10 L/min; and
further preferably, an ultraviolet radiation power is 15 W-20 W, and an irradiation distance is 20-30 cm.

Preferably, in step (2), the bacterial cells are allowed to stand and cultured in a medium;

more preferably, the medium is a YPD medium;
further preferably, the medium has a pH of 5.0-6.0;
more preferably, a culture temperature is 28-32°C; and
more preferably, the culture time is 30-60 min.

More preferably, in step (3), the recovered bacterial cells obtained in step (2) are cultured in an evolution medium containing an inhibitor, a culture solution is then transferred into a new evolution medium for culture, and the transfer is repeated for 50-150 times;

preferably, the inhibitor is one or a combination of more of 6-azauracil, 8-azaguanine, actidione, 6-mercaptopurine, diaminopurine, 5-bromouracil, and 5-fluorouracil;

more preferably, a concentration of the inhibitor in the transferred medium is increased;

further preferably, in the transfer process, the concentration of the inhibitor in the medium is increased by 50-1000 mg/L;

more preferably, after 20-30 h of culture, the transfer is performed for, preferably, 24 h;

more preferably, a culture temperature is 28-33°C, and a shake culture is performed at 150-220 rpm;

more preferably, the inhibitor concentration is 50 mg-1000 mg/L, and preferably, the inhibitor concentration is 50 mg-500 mg/L; and

more preferably, an initial concentration of the inhibitor is 50 mg -100 mg/L.

More preferably, in step (4), a mutagenic library is stained with an RNA fluorescent dye, and a strain with high fluorescence is selected to obtain the high-RNA yeast; and

more preferably, the RNA fluorescent dye is one or a combination of more of SYTO® RNASelect™, Hoechst 33258, Quant-iT™ RiboGreen® RNA Reagent, or ethidium bromide, preferably SYTO® RNASelect.

More preferably, the method further includes a step (5) of amplification verification.

More preferably, the strain sorted out in step (4) is subjected to fermentation verification in a fermenter, and the RNA content of the strain is determined.

More preferably, a fermentation medium contains the following components in percentage by mass: 2-6% of glucose, 0.5-2% of yeast extract, 0.2-0.4% of magnesium sulfate heptahydrate, 0.2-0.4% of zinc sulfate heptahydrate, 0.5-1.2% of potassium dihydrogen phosphate, and the balance of water;

more preferably, the fermentation medium has a pH of 5.0-6.0;

more preferably, a fermentation stirring speed is 300-600 rpm; and

more preferably, a fermentation aeration capacity is 3-6 L/min.

By means of the method provided by the present invention, the high-RNA yeast strain can be efficiently screened out, the dominant strain is rapidly bred, and the RNA content of the screened dominant strain can reach 15% or more. The wet weight of the bacterial cells can reach 200 g/L or more, achieving high-density fermentation of the high-RNA strain. This strain can reduce the production cost of RNA, nucleotides and other related products in the actual production process.

**Brief Description of the Drawings**

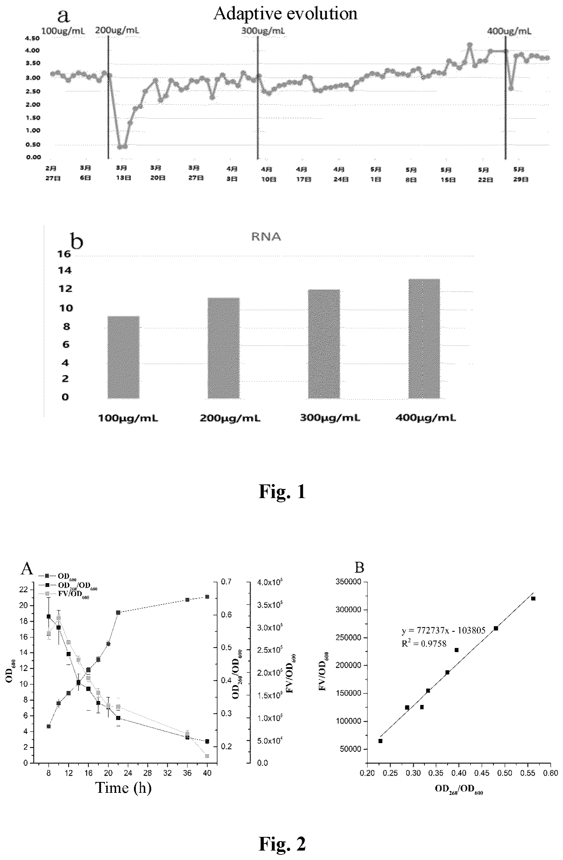

FIG. 1 shows the changes in OD600 and RNA content of bacterial cells subjected to adaptive evolution over an inhibitor concentration, wherein (a) shows the changes in OD600 of the bacterial cells subjected to adaptive evolution, and (b) shows the changes in RNA content of the bacterial cells subjected to adaptive evolution;

FIG. 2 shows a relationship between the RNA content in yeast cells and the fluorescence intensity, wherein (A) shows a relationship between the RNA content in the yeast cells and the fluorescence intensity, and (B) shows correlation analysis between the RNA content in the yeast cells and the fluorescence intensity;

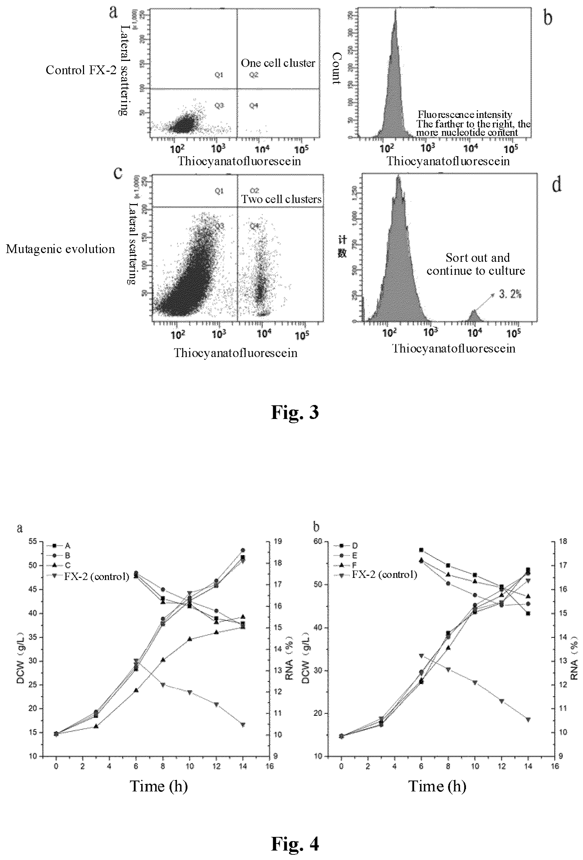

FIG. 3 shows flow cytometry sorting results, wherein (a) shows a relationship between the fluorescence intensity and lateral scattering intensity of a starting strain, (b) shows a relationship between the fluorescence intensity and the number of cells of the starting strain, (c) shows a relationship between the fluorescence intensity and lateral scattering intensity of a mutagenic evolution library, and (d) shows a relationship between the fluorescence intensity and the number of cells in the mutagenic evolution library; and

FIG. 4 shows amplification verification results of dominant strains in a 3 L fermenter, wherein (a) shows biomass and RNA results of strains obtained in Examples 1, 2 and 3 and the starting strain *Saccharomyces cerevisiae* FX-2 in the 3 L fermenter, and (b) shows biomass and RNA results of strains obtained in Examples 7, 8 and 9 and the starting strain *Saccharomyces cerevisiae* FX-2 in the 3 L fermenter.

**Detailed Description of the Invention**

According to a method for high-throughput and rapid breeding of a high-RNA yeast provided by the present invention, the RNA content is reflected by a fluorescence value, and a dominant strain with high RNA content is bred rapidly by using a flow cytometry sorting technology. High-throughput screening is a more efficient method than conventional screening. According to the method for high-throughput and rapid breeding of the high-RNA yeast provided by the present invention, in a large-scale screening process, yeast cells are first stained with an RNA dye, and the stained cells emit fluorescent light at specific wavelengths. The fluorescence intensity is positively correlated with the RNA content. The strained cells are subjected to fluorescence analysis by a flow cytometer, which can quickly screen out cells with high fluorescence intensity (i.e., high RNA content).

In a specific embodiment provided by the present invention, the high-RNA yeast is screened by a method including the following steps:

(1) establishing a microbial mutant library:

in a specific embodiment of the present invention, the microbial mutant library is obtained by mutagenesis;
in a specific embodiment of the present invention, the mutagenesis includes chemical mutagenesis, physical mutagenesis or a combination of chemical mutagenesis and physical mutagenesis;
in a specific embodiment of the present invention, a chemical mutagen is one or a combination of more of ethyl methanesulfonate, diethyl sulfate, and sodium azide;
in a specific embodiment of the present invention, an action concentration of the chemical mutagens is 1-6% (w/v), and the action time is 0.5-2 h;
in a specific embodiment of the present invention, the physical mutagenesis method includes one or a combination of two of ultraviolet radiation and atmospheric and room temperature plasma;
in a specific embodiment of the present invention, the physical mutagenesis time is 30 s-90 s;

(2) recovering bacterial cells:
in a specific embodiment of the present invention, the bacterial cells subjected to mutagenesis are allowed to stand and cultured in a medium; in a preferred embodiment of the present invention, the medium is a YPD medium having a pH of 5.0-6.0, and is allowed to stand and cultured at 30°C for 30-60 min;
(3) subjecting the mutant library to adaptive evolution:

in a specific embodiment of the present invention, the recovered bacterial cells are transferred into an evolution medium containing an inhibitor, subjected to shake culture for 20-30 h, and then transferred into a fresh evolution medium for continuous culture, and the transfer is repeated; in addition, the inhibitor concentration is continuously increased during the transfer culture;
in a specific embodiment of the present invention, the inhibitor in the evolution medium is one or a combination of more of 6-azauracil, 8-azaguanine, actidione, 6-mercaptopurine, diaminopurine, 5-bromouracil, and 5-fluorouracil;
in a specific embodiment of the present invention, an initial concentration of the inhibitor is 50 mg -100 mg/L;

(4) carrying out high-throughput screening on the evolutionary library:
in a specific embodiment of the present invention, a mutagenic library is stained with an RNA-specific fluorescent dye, each cell is subjected to fluorescence analysis by a flow cytometer after straining, and a strain with high fluorescence (i.e., high RNA content) is sorted out by using a positive correlation between the fluorescence intensity and the RNA content in yeast cells; and
(5) performing amplification verification:
in a specific embodiment of the present invention, the strain sorted out in the previous step is subjected to fermentation verification in a fermenter, and the RNA content of the strain is determined.

In a specific embodiment of the present invention, a fermentation medium contains the following components in percentage by mass: 2-6% of glucose, 0.5-2% of yeast extract, 0.2-0.4% of magnesium sulfate heptahydrate, 0.2-0.4% of zinc sulfate heptahydrate, 0.5-1.2% of potassium dihydrogen phosphate, and the balance of water, and has a pH of 5.0-6.0.

In a specific embodiment of the present invention, the stirring is performed at 300-600 rpm, followed by aeration at 3-6 L/min, and the biomass and RNA content are determined at regular intervals.

In an example of the present invention, the mutagenesis is performed by atmospheric and room temperature plasma at a mutagenesis power of 100 W and an air flow rate of 10 L/min.

In an example of the present invention, the mutagenesis is performed by ultraviolet radiation at a mutagenesis power of 15W -20W and an irradiation distance of 30 cm.

In an example of the present invention, the biomass and RNA content are detected by using the following methods:
the biomass detection method: 1. a 10 mL centrifuge tube is taken, and a weight m1 of an empty centrifuge tube is accurately weighed; 2. 10 mL of fermentation broth is taken accurately and centrifuged at 5000 rpm for 10 min, and after a supernatant is poured off, the fermentation broth is dried at 105°C to a constant weight, and a weight m2 is weighed; 3. biomass is calculated according to DCW(g/L)=(m2-m1)*100.

RNA detection method:

reagent: 1. 0.5 N perchloric acid ($HClO_4$): 400 mL of distilled water is added to 21.5 mL of 70% (or 22.1 mL of 68%) perchloric acid, and then added with distilled water to a constant volume of 500 mL.

2. 0.25 N perchloric acid ($HClO_4$): 250 mL of 0.5 N perchloric acid ($HClO_4$) is diluted to 500 mL with distilled water.

equipment: (1) milligram balance; (2) 4000 rpm centrifuge, which can separate yeast; (3) centrifuge tube, with a capacity of at least 10 mL; (4) spectrophotometer (260 nm); (5) 70°C hot water bath; (6) 4°C cold water bath; (7) 10 mL and 1 mL pipettes; and (8) 100 mL volumetric flask.

Method:

1. if it is a dry yeast powder, 0.06-0.15 g of yeast is weighed and added to a centrifuge tube; if it is 18% yeast milk, 0.4-0.8 g of yeast milk is weighed; and if it is 3.5% liquid, 1.5-3.0 g of liquid is weighed.

2. 8 mL of cold 0.25 N $HClO_4$ is added to a centrifuge tube and treated in a 4°C water bath for 15 min.

3. Centrifugation is performed at 4000 rpm for 10 min.

4. A substance floating on the surface is gently poured off, 5 mL of 0.5 N $HClO_4$ was added, shaken for mixing well, and treated in a 70°C water bath for 15 min with shaking every 3-4 min. Centrifugation is performed at 4000 rpm for 10 min, and 1 mL of supernatant is pipetted, and added with distilled water to a constant volume of 100 mL.

5. The absorbance is determined at 260 nm with distilled water as a blank control.

A calculation formula is: 
$$\%RNA = \frac{\text{absorbance} \times \text{dilution factor} \times 0.03365 \times 5}{\text{sample mass} \times \text{percentage of solids in sample}} \times 100\%$$
in dry yeast.

In the present invention, during the calculation of the RNA content in dry yeast, the sample weight is calculated in milligrams. For example, the weight of the weighed dry yeast is 145 mg, the absorbance is 0.7, the solid content is 96%, and the RNA content in the obtained dry yeast is calculated as follows: 
$$\%RNA = \frac{0.7 \times 100 \times 0.03365 \times 5}{145mg \times 0.96} \times 100\%$$
.

Explanations of the relevant terms involved in the present invention are as follows:

OD260/OD600 refers to the RNA content per unit bacterial cell;
FV/OD600 refers to the fluorescence intensity per unit bacterial cell (after staining); and DCW refers to a dry cell weight.

Source of reagents and instruments used in the examples below are shown in Table 1.

Table 1

| Substances | Performance indexes | Sold by manufacturers or from publicly available sources |
|---|---|---|
| YPD medium | Reagent grade | Aobox Biotechnology (Beijing, China) |
| YNB medium | Reagent grade | Angel Yeast Co., Ltd. |
| 6-azauracil | Purity of 99% | Macklin reagent |
| 8-azaguanine | Purity of 98% | Macklin reagent |
| Glucose | Analytically pure | Macklin reagent |
| Magnesium sulfate heptahydrate | Analytically pure | Macklin reagent |
| Zinc sulfate heptahydrate | Analytically pure | Macklin reagent |
| Potassium dihydrogen phosphate | Analytically pure | Macklin reagent |

(continued)

| Substances | Performance indexes | Sold by manufacturers or from publicly available sources |
|---|---|---|
| Ethyl methanesulfonate | Purity of 99% | Macklin reagent |
| Diethyl sulfate | Purity of 99% | Macklin reagent |
| Sodium azide | Purity of 99% | Macklin reagent |
| Phosphate buffer | pH of 7.2 | Macklin reagent |
| Actidione | Purity of >95% | Aladdin reagent |
| 6-mercaptopurine | Purity of >99% | Aladdin reagent |
| Diaminopurine | Purity of >98% | Aladdin reagent |
| 5-bromouracil | Purity of >99% | Aladdin reagent |
| 5-fluorouracil | Purity of >99% | Aladdin reagent |
| SYTO ® RNASelect™ | 5 mM DMSO solution | Thermo Fisher |
| Hoechst 33258 | Concentration of 10 mM | AbMole |
| Quant-iT™ RiboGreen® RNA Reagent | DMSO solution | Beijing NovoBiotechnology Co., Ltd. |
| Ethidium bromide | 10 mg/mL | Thermo Fisher |

*Saccharomyces cerevisiae* FX-2 used in the present invention was preserved in the China Center for Type Culture Collection (CCTCC) on August 1, 2016, and had a preservation number of CCTCC NO: M2016418. This strain was documented in the patent publication text with publication number CN108220175A.

**Example 1**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate (EMS), mixed well, shaken at 30°C, 50 rpm for 30 min, and then centrifuged; and a bacterial cell precipitate was washed twice.

Recovery of bacterial cells: the above-mentioned bacterial cell precipitate was resuspended in 5 mL of YPD medium e, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 100 mg of 6-azauracil were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 50 times; and the inhibitor concentration was continuously increased to 200 mg/L, 300 mg/L, 400 mg/L, and 500 mg/L. After the evolution was completed, a bacterial solution obtained from the last culture was taken, and the bacterial cells were collected by centrifugation. The changes in OD600 and RNA content of the obtained bacterial cells over an inhibitor concentration were shown in FIG. 1, wherein (a) showed the changes in OD600 of the bacterial cells subjected to adaptive evolution, and (b) showed the changes in RNA content of the bacterial cells subjected to adaptive evolution. As can be seen from FIG. 1, the OD600 value and RNA content of the bacterial cells increased with the increase in inhibitor concentration.

High-throughput screening of an evolutionary library: a 500 nM staining solution was prepared by diluting SYTO ® RNASelect™ in 10000 folds; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

A relationship between the RNA content in yeast cells and the fluorescence intensity was shown in FIG. 2, wherein (a) showed a relationship between the RNA content in the yeast cells and the fluorescence intensity, and (b) showed correlation analysis between the RNA content in the yeast cells and the fluorescence intensity. As can be seen from FIG. 2, the RNA content in the yeast cells was linearly correlated with the fluorescence intensity.

Flow cytometry sorting results were shown in FIG. 3, wherein (a) showed a relationship between the fluorescence intensity and lateral scattering intensity of a starting strain, (b) showed a relationship between the fluorescence intensity and the number of cells of the starting strain, (c) showed a relationship between the fluorescence intensity and lateral scattering intensity of a mutagenic evolution library, and (d) showed a relationship between the fluorescence intensity and the number of cells in the mutagenic evolution library. As can be seen in FIG. 3, the evolved cell library was sorted by the flow cytometer and showed that one cell cluster had fluorescence higher than other cells.

Amplification verification: the above-mentioned dominant strain A screened by the flow cytometer was subjected to fermentation verification in a 3L fermenter, wherein a fermentation medium included the following components in percentage by mass: 6% of glucose, 2% of yeast extract, 0.4% of magnesium sulfate heptahydrate, 0.4% of zinc sulfate heptahydrate, 1.2% of potassium dihydrogen phosphate, and the balance of water and had a pH of 5.5; the stirring was performed at 600 rpm, followed by aeration at 4 L/min; and the biomass and RNA content were determined at regular intervals. After the fermentation was completed, the RNA content reached 15.2%, and the dry cell weight reached 51.7 g/L.

The amplification verification results of the dominant strain A in the 3 L fermenter were shown in FIG. 4. As can be seen from FIG. 4, the RNA content of the screened dominant strain A after the fermentation culture in the 3 L fermenter was significantly higher than that of the starting strain *Saccharomyces cerevisiae* FX-2.

**Example 2**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 3% (3g by mass of sodium azide contained in every 100 mL of solution) sodium azide, mixed well, shaken at 30°C, 50 rpm for 60 min, and then centrifuged; and a bacterial cell precipitate was washed twice.

Recovery of bacterial cells:the above-mentioned bacterial cell precipitate was resuspended in 5 mL of YPD medium was resuspended , allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 50 mg of 8-azaguanine were prepared into 1 L of evolution medium solution, followed by 0.22 $\mu$m filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 100 times; and the inhibitor concentration was continuously increased to 100 mg/L, 150 mg/L, 200 mg/L, 250 mg/L, and 300 mg/L. After the evolution was completed, a bacterial solution obtained from the last culture was taken, and the bacterial cells were collected by centrifugation.

High-throughput screening of an evolutionary library: a 500 nM staining solution was prepared by diluting Quant-iT™ RiboGreen® RNA Reagent; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain B screened by the flow cytometer was subjected to fermentation verification in a 3 L fermenter, wherein a fermentation medium included the following components in percentage by mass: 2% of glucose, 0.5% of yeast extract, 0.4% of magnesium sulfate heptahydrate, 0.4% of zinc sulfate heptahydrate, 1.2% of potassium dihydrogen phosphate, and the balance of water and had a pH of 6.0; the stirring is performed at 400 rpm, followed by aeration at 4 L/min; and the biomass and RNA content are determined at regular intervals. After the fermentation was completed, the RNA content reached 15.1%, and the dry cell weight reached 53.2 g/L.

The amplification verification results of the dominant strain B in the 3 L fermenter were shown in FIG. 4. As can be seen from FIG. 4, the RNA content of the screened dominant strain B after the fermentation culture in the 3 L fermenter was significantly higher than that of the starting strain *Saccharomyces cerevisiae* FX-2.

**Example 3**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min;

bacterial cells were collected; a bacterial cell precipitate was washed twice with phosphate buffer, added with 1 mL of prepared 3% (3 g by mass of diethyl sulfate contained in every 100 mL of solution) diethyl sulfate, mixed well, shaken at 30°C, 50 rpm for 60 min, subjected to mutagenesis for 60 s under a condition of atmospheric and room temperature plasma (ARTP), and then centrifuged; and a bacterial cell precipitate was washed twice.

Recovery of bacterial cells: the above-mentioned bacterial cell precipitate was resuspended in 5 mL of YPD medium, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 50 mg of actidione were prepared into 1 L of evolution medium solution, followed by 0.22 $\mu$m filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 150 times; and the inhibitor concentration was continuously increased to 200 mg/L, 300 mg/L, 400 mg/L, and 500 mg/L. After the evolution was completed, a bacterial solution obtained from the last culture was taken, and the bacterial cells were collected by centrifugation.

High-throughput screening of an evolutionary library: a 500 nM staining solution was prepared by diluting SYTO® RNASelect™ in 10000 folds; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain C screened by the flow cytometer was subjected to fermentation verification in a 3L fermenter, wherein a fermentation medium included the following components in percentage by mass: 4% of glucose, 1% of yeast extract, 0.2% of magnesium sulfate heptahydrate, 0.2% of zinc sulfate heptahydrate, 1% of potassium dihydrogen phosphate, and the balance of water and had a pH of 5.0; the stirring is performed at 600 **rpm,** followed by aeration at 6 L/min; and the biomass and RNA content are determined at regular intervals. After the fermentation was completed, the RNA content reached 15.5%, and the dry cell weight reached 37.1 g/L.

The amplification verification results of the dominant strain C in the 3 L fermenter were shown in FIG. 4. As can be seen from FIG. 4, the RNA content of the screened dominant strain C after the fermentation culture in the 3 L fermenter was significantly higher than that of the starting strain *Saccharomyces cerevisiae* FX-2.

## Example 4

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate, mixed well, shaken at 30°C, 50 rpm for 30 min, then subjected to mutagenesis for 30 s under a condition of ultraviolet radiation, and centrifuged; and a bacterial cell precipitate was washed twice.

Recovery of bacterial cells: the above-mentioned bacterial cell precipitate was resuspended in 5 mL of YPD medium , allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 50 mg of 6-mercaptopurine were prepared into 1 L of evolution medium solution, followed by 0.22 $\mu$m filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 60 times; and the inhibitor concentration was continuously increased to 100 mg/L, 150 mg/L, 200 mg/L, 250 mg/L, and 300 mg/L. After the evolution was completed, a bacterial solution obtained from the last culture was taken, and the bacterial cells were collected by centrifugation.

High-throughput screening of an evolutionary library: ethidium bromide was diluted into a 1 ug/mL staining solution; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 **rpm** for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain screened by the flow cytometer was subjected to fermentation verification in a 3L fermenter, wherein a fermentation medium included the following components in percentage by mass: 2% of glucose, 0.5% of yeast extract, 0.2% of magnesium sulfate heptahydrate, 0.2% of zinc sulfate heptahydrate, 0.5% of potassium dihydrogen phosphate, and the balance of water and had a pH of 5.0; the stirring was performed at 300 rpm, followed by aeration at 3 L/min; and the biomass and RNA content are determined at regular intervals. After the fermentation was completed, the RNA content reached 15.3%, and the dry cell weight reached 52.9 g/L.

## Example 5

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate (EMS), mixed well, shaken at 30°C, 50 rpm for 30 min, and then centrifuged; and a bacterial cell precipitate was washed twice.

Recovery of bacterial cells: the above-mentioned bacterial cell precipitate was resuspended in 5 mL of YPD medium, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 100 mg of diaminopurine were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 120 times; and the inhibitor concentration was continuously increased to 200 mg/L, 300 mg/L, 400 mg/L, and 500 mg/L. After the evolution was completed, a bacterial solution obtained from the last culture was taken, and the bacterial cells were collected by centrifugation.

High-throughput screening of an evolutionary library: Hoechst 33258 was diluted into a 1 uM staining solution; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain screened by the flow cytometer was subjected to fermentation verification in a 3 L fermenter, wherein a fermentation medium included the following components in percentage by mass: 6% of glucose, 2% of yeast extract, 0.4% of magnesium sulfate heptahydrate, 0.4% of zinc sulfate heptahydrate, 1.2% of potassium dihydrogen phosphate, and the balance of water and had a pH of 5.5; the stirring is performed at 600 rpm, followed by aeration at 4 L/min; and the biomass and RNA content are determined at regular intervals. After the fermentation was completed, the RNA content reached 15.2%, and the dry cell weight reached 51.7 g/L.

## Example 6

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 3% (3 g by mass of sodium azide contained in every 100 mL of solution) sodium azide, mixed well, shaken at 30°C, 50 rpm for 60 min, and then centrifuged; and a bacterial cell precipitate was washed twice.

Recovery of bacterial cells: the above-mentioned bacterial cell precipitate was resuspended in 5 mL of YPD medium was resuspended , allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 50 mg of 5-bromouracil were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 90 times; and the inhibitor concentration was continuously increased to 100 mg/L, 150 mg/L, 200 mg/L, 250 mg/L, and 300 mg/L. After the evolution was completed, a bacterial solution obtained from the last culture was taken, and the bacterial cells were collected by centrifugation.

High-throughput screening of an evolutionary library: a 500 nM staining solution was prepared by diluting Quant-iT™ RiboGreen® RNA Reagent; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain screened by the flow cytometer was subjected to fermentation verification in a 3 L fermenter, wherein a fermentation medium included the following components in percentage by mass: 2% of glucose, 0.5% of yeast extract, 0.4% of magnesium sulfate heptahydrate, 0.4% of zinc sulfate heptahydrate, 1.2% of potassium dihydrogen phosphate, and the balance of water and had a pH of 6.0; the stirring was performed at 400 rpm, followed by aeration at 4 L/min; and the biomass and RNA content are determined at regular intervals. After the fermentation was completed, the RNA content reached 15.4%, and the dry cell weight reached 53.2 g/L.

**Example 7**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, subjected to mutagenesis for 60 s under a condition of atmospheric and room temperature plasma (ARTP), and then centrifuged; and a bacterial cell precipitate was washed twice.

Recovery of bacterial cells:the above-mentioned bacterial cell precipitate was resuspended in 5 mL of YPD medium, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, and 50 mg of 5-fluorouracil were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 130 times; and the inhibitor concentration was continuously increased to 100 mg/L, 150 mg/L, 200 mg/L, 250 mg/L, and 300 mg/L. After the evolution was completed, a bacterial solution obtained from the last culture was taken, and the bacterial cells were collected by centrifugation.

High-throughput screening of an evolutionary library: ethidium bromide was diluted into a 1 ug/mL staining solution; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 **rpm** for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain D screened by the flow cytometer was subjected to fermentation verification in a 3L fermenter, wherein a fermentation medium included the following components in percentage by mass: 6% of glucose, 2% of yeast extract, 0.2% of magnesium sulfate heptahydrate, 0.2% of zinc sulfate heptahydrate, 1% of potassium dihydrogen phosphate, and the balance of water and had a pH of 5.5; the stirring was performed at 400 **rpm,** followed by aeration at 3 L/min; and the biomass and RNA content are determined at regular intervals. After the fermentation was completed, the RNA content reached 15.0%, and the dry cell weight reached 53.5 g/L.

The amplification verification results of the dominant strain D in the 3 L fermenter were shown in FIG. 4. As can be seen from FIG. 4, the RNA content of the screened dominant strain D after the fermentation culture in the 3 L fermenter was significantly higher than that of the starting strain *Saccharomyces cerevisiae* FX-2.

**Example 8**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 3% (3 g by mass of diethyl sulfate contained in every 100 mL of solution) diethyl sulfate, mixed well, shaken at 30°C, 50 rpm for 60 min, subjected to mutagenesis for 60 s under a condition of atmospheric and room temperature plasma (ARTP), and then centrifuged; and a bacterial cell precipitate was washed twice.

Recovery of bacterial cells: the above-mentioned bacterial cell precipitate was resuspended in 5 mL of YPD mediume, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, 25 mg of 8-azaguanine, and 25 mg of actidione were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 50 times; and the inhibitor concentration was continuously increased to 100 mg/L, 150 mg/L, 200 mg/L, 250 mg/L, and 300 mg/L. After the evolution was completed, a bacterial solution obtained from the last culture was taken, and the bacterial cells were collected by centrifugation.

High-throughput screening of an evolutionary library: Hoechst 33258 was diluted into a 1 uM staining solution; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain E screened by the flow cytometer was subjected to fermentation verification in a 3 L fermenter, wherein a fermentation medium included the following components in percentage by mass: 4% of glucose, 1% of yeast extract, 0.2% of magnesium sulfate heptahydrate, 0.2% of zinc sulfate heptahydrate, 1% of potassium dihydrogen phosphate, and the balance of water and had a pH of 5.0; the stirring was performed at 600 rpm, followed by aeration at 6 L/min; and the biomass and RNA content were determined at regular intervals. After the fermentation was completed, the RNA content reached 15.4%, and the dry cell weight reached 52.6 g/L.

The amplification verification results of the dominant strain E in the 3 L fermenter were shown in FIG. 4. As can be seen from FIG. 4, the RNA content of the screened dominant strain E after the fermentation culture in the 3 L fermenter was significantly higher than that of the starting strain *Saccharomyces cerevisiae* FX-2.

**Example 9**

Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate, mixed well, shaken at 30°C, 50 rpm for 30 min, then subjected to mutagenesis for 30 s under a condition of ultraviolet radiation, and centrifuged; and a bacterial cell precipitate was washed twice.

Recovery of bacterial cells: the above-mentioned bacterial cell precipitate was resuspended in 5 mL of YPD mediume, allowed to stand and cultured at 30°C for 60 min, and then centrifuged at 5000 rpm for 5 min, and bacterial cells were collected.

Adaptive evolution of a mutagenic library: 6.7 g of YNB, 20 g of ammonium sulfate, 40 mg of 5-fluorouracil, and 40 mg of 6-mercaptopurine were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 100 times; and the inhibitor concentration was continuously increased to 100 mg/L, 200 mg/L, 300 mg/L, 400 mg/L, and 500 mg/L. After the evolution was completed, a bacterial solution obtained from the last culture was taken, and the bacterial cells were collected by centrifugation.

High-throughput screening of an evolutionary library: a 500 nM staining solution was prepared by diluting SYTO® RNASelect™ in 10000 folds; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm. After the analysis with the flow cytometer, an evolved cell library showed that one cell cluster had fluorescence significantly higher than other cells.

Amplification verification: the above-mentioned dominant strain F screened by the flow cytometer was subjected to fermentation verification in a 3 L fermenter, wherein a fermentation medium included the following components in percentage by mass: 2% of glucose, 0.5% of yeast extract, 0.2% of magnesium sulfate heptahydrate, 0.2% of zinc sulfate heptahydrate, 0.5% of potassium dihydrogen phosphate, and the balance of water and had a pH of 5.0; the stirring was performed at 300 rpm, followed by aeration at 3 L/min; and the biomass and RNA content are determined at regular intervals. After the fermentation was completed, the RNA content reached 15.7%, and the dry cell weight reached 52.9 g/L.

The amplification verification results of the dominant strain F in the 3 L fermenter were shown in FIG. 4. As can be seen from FIG. 4, the RNA content of the screened dominant strain F after the fermentation culture in the 3 L fermenter was significantly higher than that of the starting strain *Saccharomyces cerevisiae* FX-2.

**Comparative example 1**

1) Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate (EMS), mixed well, shaken at 30°C, 50 rpm for 30 min, and then centrifuged; and a bacterial cell precipitate was washed twice.

2) Recovery of bacterial cells: the above-mentioned bacterial cell precipitate was resuspended in 5 mL of YPD medium, allowed to stand and cultured at 30°C for 60 min, and then centrifuged at 5000 rpm for 5 min, and bacterial cells were collected.

3) High-throughput screening of an evolutionary library: a 500 nM staining solution was prepared by diluting SYTO ® RNASelect™ in 10000 folds; 1 mL of staining solution was pipetted, and added to the bacterial cells obtained by centrifugation after the above-mentioned evolution; the bacterial cells were resuspended, subjected to shake incubation at 30°C, 50 rpm for 20 min, and centrifuged at 5000 rpm for 5 min; the bacterial cells were collected; a precipitate was washed twice with phosphate buffer, and resuspended; and the resuspension solution was subjected to fluorescence analysis by a flow cytometer, and the sorting conditions were as follows: an excitation wavelength of 490 nm, and an emission wavelength of 530 nm.

4) Amplification verification: the above-mentioned dominant strain screened by the flow cytometer was subjected to fermentation verification in a 3 L fermenter, wherein a fermentation medium included the following components in percentage by mass: 6% of glucose, 2% of yeast extract, 0.4% of magnesium sulfate heptahydrate, 0.4% of zinc sulfate heptahydrate, 1.2% of potassium dihydrogen phosphate, and the balance of water and had a pH of 5.5; the stirring was performed at 600 rpm, followed by aeration at 4 L/min; and the biomass and RNA content were determined at regular intervals. After the fermentation was completed, the RNA content reached 11.8%, and the dry cell weight reached 52.3 g/L.

**Comparative example 2**

1) Mutagenesis of a starting strain: *Saccharomyces cerevisiae* FX-2 was subjected to shake culture in 100 mL of YPD medium at 30°C, 200 rpm for 24 h; 1 mL of bacterial solution was pipetted, and centrifuged at 12000 rpm for 1 min; bacterial cells were collected; a bacterial cell precipitate was washed twice with phosphate buffer, added with 1 mL of prepared 5% (5 g by mass of ethyl methanesulfonate contained in every 100 mL of solution) ethyl methanesulfonate (EMS), mixed well, shaken at 30°C, 50 rpm for 30 min, and then centrifuged; and a bacterial cell precipitate was washed twice.

2) Recovery of bacterial cells: the above-mentioned bacterial cell precipi bacterial celltate was resuspended in 5 mL of YPD medium, allowed to stand and cultured at 30°C for 60 min, and then centrifuged at 5000 rpm for 5 min, and bacterial cells were collected.

3) Adaptive evolution of a mutagenic library: 6.7 g of YNB, 20 g of ammonium sulfate, and 100 mg of 6-azauracil were prepared into 1 L of evolution medium solution, followed by 0.22 μm filter sterilization; 50 mL of evolution medium was taken to resuspend the above-mentioned bacterial cells, and subjected to shake culture at 30°C, 200 rpm for 24 h; 0.5 mL of culture solution was pipetted and transferred into a fresh evolution medium, and continuously cultured for 24 h, and the transfer was repeated for 50 times; and the inhibitor concentration was continuously increased to 200 mg/L, 300 mg/L, 400 mg/L, and 500 mg/L. After the evolution was completed, a bacterial solution obtained from the last culture was taken, and the bacterial cells were collected by centrifugation.

4) Screening of evolutionary library: the bacterial cells obtained by centrifugation after the above-mentioned evolution were suspended, subjected to shake incubation at 30°C, 50 rpm for 20 min, and centrifuged at 5000 rpm for 5 min; a precipitate was washed twice with phosphate buffer and resuspended; the resuspension solution was diluted to an appropriate concentration and coated on a YPD solid plate, and allowed to stand and cultured at 30°C for 24 h; and then single colonies were picked, and the RNA contents of the single colonies were determined one by one.

5) Amplification verification: a strain with high RNA content was subjected to fermentation verification in a 3 L fermenter, wherein a fermentation medium included the following components in percentage by mass: 6% of glucose, 2% of yeast extract, 0.4% of magnesium sulfate heptahydrate, 0.4% of zinc sulfate heptahydrate, 1.2% of potassium dihydrogen phosphate, and the balance of water and had a pH of 5.5; the stirring was performed at 600 rpm, followed by aeration at 4 L/min; and the biomass and RNA content were determined at regular intervals. After the fermentation was completed, the RNA content reached 12.6%, and the dry cell weight reached 51.6 g/L.

The foregoing descriptions are merely preferred examples of the present invention, and are not intended to limit the present invention in any form. Any modifications, equivalent substitutions, improvements, etc. made within the spirit and principles of the present invention should fall within the protection scope of the present invention.

**Claims**

1. A method for the high-throughput and rapid breeding of a high-RNA yeast, **characterized by** comprising the following steps:

   (1) establishing a microbial mutant library;
   (2) recovering bacterial cells obtained in step (1);
   (3) subjecting the mutant library to adaptive evolution; and
   (4) carrying out high-throughput screening on the evolutionary library to obtain a high-RNA yeast.

2. The method according to claim 1, **characterized in that** in step (1), the establishing the microbial mutant library is performed by using chemical mutagenesis, physical mutagenesis or a combination of chemical mutagenesis and physical mutagenesis.

3. The method according to claim 2, **characterized in that** a chemical mutagen is one or a combination of more of ethyl methanesulfonate, diethyl sulfate, and sodium azide;

   preferably, an action concentration of the chemical mutagen is 1-6% (w/v); and
   more preferably, the action time is 0.5-2 h.

4. The method according to claim 2, **characterized in that** the physical mutagenesis is performed by using one or a combination of two of ultraviolet radiation and atmospheric and room temperature plasma; preferably, the physical mutagenesis time is 30 s-90 s;

   preferably, the atmospheric and room temperature plasma has a power of 80-120 W, and an air flow rate of 8-15 L/min; more preferably, the atmospheric and room temperature plasma has a power of 100 W, and an air flow rate of 10 L/min; and
   further preferably, an ultraviolet radiation power is 15 W-20 W, and an irradiation distance is 20-30 cm.

5. The method according to any one of claims 1 to 4, **characterized in that** in step (2), the bacterial cells are allowed to stand and cultured in a medium;

   preferably, the medium is a YPD medium;
   more preferably, the medium has a pH of 5.0-6.0;
   further preferably, a culture temperature is 28-32°C; and
   more preferably, the culture time is 30-60 min.

6. The method according to any one of claims 1 to 5, **characterized in that** in step (3), the recovered bacterial cells obtained in step (2) are cultured in an evolution medium containing an inhibitor, a culture solution is then transferred into a new evolution medium for culture, and the transfer is repeated for 50-150 times;

   preferably, the inhibitor is one or a combination of more of 6-azauracil, 8-azaguanine, actidione, 6-mercaptopurine, diaminopurine, 5-bromouracil, and 5-fluorouracil;
   more preferably, a concentration of the inhibitor in the transferred medium is increased;
   further preferably, in the transfer process, the concentration of the inhibitor in the medium is increased by 50-1000 mg/L;

more preferably, after 20-30 h of culture, the transfer is performed for, preferably, 24 h;
more preferably, a culture temperature is 28-33°C, and a shake culture is performed at 150-220 rpm;
more preferably, the inhibitor concentration is 50 mg-1000 mg/L, and preferably, the inhibitor concentration is 50 mg-500 mg/L; and
more preferably, an initial concentration of the inhibitor is 50 mg -100 mg/L.

7. The method according to any one of claims 1 to 6, **characterized in that** in step (4), a mutagenic library is stained with an RNA fluorescent dye, and a strain with high fluorescence is selected to obtain the high-RNA yeast; and
preferably, the RNA fluorescent dye is one or a combination of more of SYTO ® RNASelect™, Hoechst 33258, Quant-iT™ RiboGreen® RNA Reagent, or ethidium bromide, preferably SYTO ®RNASelect.

8. The method according to any one of claims 1 to 7, **characterized in that** the method further comprises a step (5) of amplification verification.

9. The method according to claim 8, **characterized in that** in step (5), the strain sorted out in step (4) is subjected to fermentation verification in a fermenter, and the RNA content of the strain is determined.

10. The method according to claim 9, **characterized in that** a fermentation medium contains the following components in percentage by mass: 2-6% of glucose, 0.5-2% of yeast extract, 0.2-0.4% of magnesium sulfate heptahydrate, 0.2-0.4% of zinc sulfate heptahydrate, 0.5-1.2% of potassium dihydrogen phosphate, and the balance of water;

preferably, the fermentation medium has a pH of 5.0-6.0;
more preferably, a fermentation stirring speed is 300-600 rpm; and
further preferably, a fermentation aeration capacity is 3-6 L/min.

Fig. 1

Fig. 2

**Fig. 3**

**Fig. 4**

**EP 4 700 123 A2**

**Patent documents cited in the description**

- CN 108220175 A